# FASCICULE DE BREVET EUROPEEN

(11) **EP 1 859 659 B1**
(45) Date de publication et mention de la délivrance du brevet: **02.05.2012**
(21) Numéro de dépôt: 06709113.2
(22) Date de dépôt: 18.01.2006
(51) Int. Cl.: H05G 1/10

(54) **DISPOSITIF DE CONTROLE DE LA DUREE D'EMISSION DES RAYONS X DANS UN DISPOSITIF DE RADIOLOGIE DENTAIRE**
VORRICHTUNG ZUR STEUERUNG DER DAUER VON RÖNTGENSTRAHLUNG BEI EINER DENTALRADIOLOGIEVORRICHTUNG
DEVICE FOR CONTROLLING THE DURATION OF THE EMISSION OF X-RAYS IN A DENTAL RADIOLOGY DEVICE

(30) Priorité: 09.02.2005 FR 0501290
(43) Date de publication de la demande: 28.11.2007
(73) Titulaire: Sopro, 13705 La Ciotat Cedex (FR)
(72) Inventeur: MAZUIR, Alain, F-83470 Saint Maximin Sainte Baume (FR)
(74) Mandataire: Domange, Maxime
(86) Numéro de dépôt international: PCT/FR2006/000106
(87) Numéro de publication internationale: WO 2006/084967

(56) Documents cités:
- US-A- 4 082 953
- US-A- 4 618 974
- US-A- 5 058 147
- US-A- 5 331 166
- US-A- 5 631 943

## Description

### Arrière-plan de l'invention

La présente invention se rapporte à un système d'acquisition d'image radiologique dentaire, à un générateur de rayons X et à un module de commande d'un capteur intra buccal pouvant être utilisé dans un tel système.

De façon connue, un système d'acquisition d'image radiologique dentaire se compose principalement d'un générateur de rayons X et d'un capteur intra buccal sensible à ces rayons X, associé à un module de commande, pour acquérir l'image radiologique.

De façon obligatoire, le générateur de rayons X est équipé d'un moyen de commande, généralement un bouton, permettant à l'opérateur de déclencher l'émission des rayons X de façon manuelle et volontaire.

Ces générateurs de rayons X sont également équipés d'une minuterie par laquelle l'opérateur peut régler une durée prédéterminée d'émission des rayons X, étant bien entendu qu'il peut volontairement arrêter l'émission par les moyens de commande précités.

Dans un premier système connu, le démarrage et la fin de l'acquisition par le capteur sont commandés par le générateur.

Un autre système connu, notamment décrit dans le document US 5,331,166 propose d'intégrer des pastilles sensibles aux rayons X dans le capteur intra buccal, ces pastilles étant adaptées à transmettre un signal au module de commande du capteur qui contrôle le démarrage et l'arrêt de l'acquisition d'images numériques en fonction de l'intensité de ce signal.

On connaît également, notamment par la divulgation du document US 5,694,448, un système d'imagerie dans lequel on compare en permanence le niveau de sortie d'un capteur CCD pour détecter, Lorsque ce niveau devient supérieur à un niveau de bruit, l'émission des rayons X par le générateur. Lorsque cette émission est détectée, l'acquisition d'images à proprement parler est effectuée.

Etant donné que le capteur CCD est lu et vidé en permanence, de façon cyclique, pour effectuer la comparaison avec le niveau de bruit, il peut se produire un cas très défavorable pendant lequel aucune image n'est acquise bien que l'émission des rayons X par le générateur ait commencé.

En pratique, de 10 à 20% des rayons X diffusés par le générateur peuvent être perdus, ce qui est bien entendu préjudiciable pour le patient. Le document US-B-6 404 854 décrit un système selon le préambule de la revendication 1.

En résumé, aucun des systèmes connus précités ne permet d'optimiser la quantité de rayons X reçue par le patient car le praticien ne peut régler la minuterie que de façon intuitive. En pratique, il préfère utiliser une surdose de rayons X afin d'être sûr d'obtenir une image de bonne qualité.

### Objet et résumé de l'invention

La présente invention a donc pour but principal de pallier de tels inconvénients en proposant un système d'acquisition d'image radiologique dentaire comportant :
- un générateur de rayons X comportant des moyens de déclenchement de l'émission des rayons X, une minuterie permettant à un opérateur de régler une durée prédéterminée d'émission des rayons X, et des moyens pour arrêter automatiquement l'émission à l'issue de la durée prédéterminée ; et
- un capteur intra buccal sensible aux rayons X, associé à un module de commande du capteur, et adapté à acquérir l'image pendant ladite émission. Dans ce système :
- le module comporte des moyens pour envoyer, selon au moins un critère prédéterminé, une commande de contrôle au générateur pour interdire, arrêter ou modifier la puissance de l'émission des rayons X avant la fin de la durée prédéterminée, de manière à contrôler la quantité de rayons X émise par le générateur ; et
- le générateur comporte des moyens de réception de la commande d'arrêt, les moyens d'arrêt du générateur étant adaptés à interdire, arrêter ou modifier la puissance d'émission sur réception de la commande.

Ainsi, conformément à l'invention, les rayons X ne sont diffusés par le générateur que pendant la phase d'intégration de l'image numérique par le capteur intra buccal.

L'invention vise également un générateur de rayons X et un module de commande d'un capteur intra buccal pouvant être utilisés dans un tel système.

Conformément à l'invention, le module possède des moyens pour interrompre ou interdire l'émission des rayons X selon des critères prédéterminés qui seront explicités ci-dessous.

Bien entendu, lorsque aucun de ces critères n'est rempli, l'émission des rayons X est interrompue par le générateur à la fin de la durée prédéterminée réglée par l'opérateur. On notera qu'un tel générateur est conforme aux réglementations qui imposent seulement que l'émission des rayons X s'arrête automatiquement d'une manière programmée au départ par l'opérateur.

Nous allons maintenant expliciter un certain nombre de conditions, ou critères, utilisées par le module de commande pour interrompre ou interdire, à distance, l'émission de rayons X par le générateur.

Bien entendu, ces critères ne sont pas exclusifs les uns des autres.

Dans un premier mode de réalisation, le module envoie la commande de contrôle sur détection d'un dysfonctionnement du capteur intra buccal.

Dans un deuxième mode de réalisation, le module possède des moyens pour détecter le type de capteur intra buccal auquel il est raccordé et des moyens pour déterminer la durée d'émission de rayons X appropriée en fonction du type de ce capteur. La commande de contrôle du générateur est alors envoyée à la fin de cette durée.

Dans un troisième mode de réalisation, le module de commande détermine une durée d'émission des rayons X en fonction du type de résolution souhaité pour l'image radiologique, et envoie la commande de contrôle au générateur à la fin de cette durée.

Dans ce mode de réalisation, le niveau de résolution peut être obtenu par le module de commande à partir d'un dispositif distant, par exemple un ordinateur. La résolution de l'image peut aussi être programmée au niveau du module lui-même.

Dans un quatrième mode de réalisation, le module comporte des moyens pour mesurer le gain moyen d'une séquence d'images précédemment acquises, et des moyens pour déterminer une quantité d'émission de rayons X ajustée en fonction de ce gain moyen et d'un gain souhaité prédéterminé. Dans ce mode de réalisation, la commande de contrôle est envoyée au générateur pour modifier la quantité de rayons X émise par le générateur conformément à cette quantité ajustée.

### Brève description des dessins

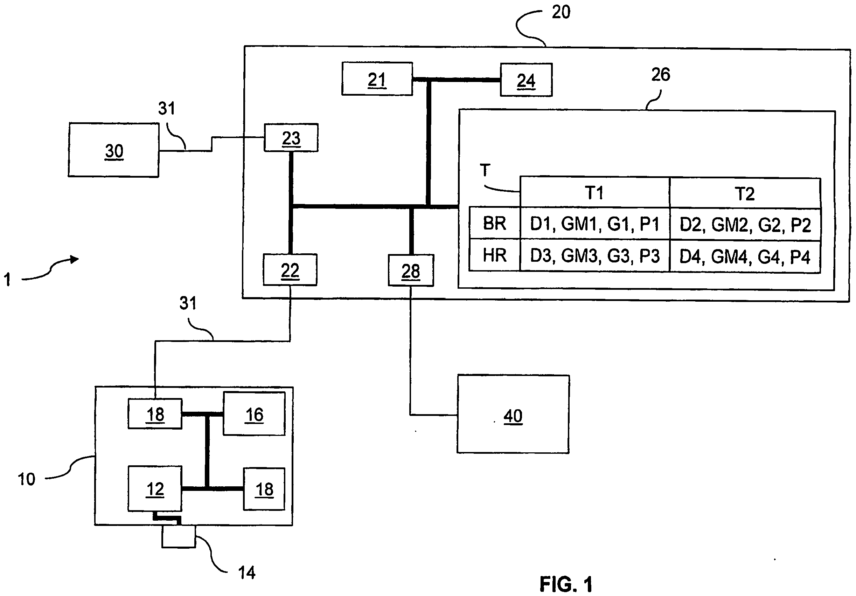

D'autres caractéristiques et avantages de la présente invention ressortiront de la description faite ci-dessous, en référence à la figure 1 annexée qui en illustre un exemple de réalisation dépourvu de tout caractère limitatif.

Cette **figure 1** représente, de façon schématique, un système 1 d'acquisition d'image radiologique dentaire conforme à l'invention.

Ce système 1 comporte un générateur 10 de rayons X conforme à l'invention dans un mode préféré de réalisation.

Ce générateur 10 de rayons X comporte une source 16 de rayons X et des moyens 14, par exemple un bouton, pour déclencher ou arrêter manuellement l'émission des rayons X par la source 16.

Le générateur 10 comporte également une minuterie 18 avec laquelle l'opérateur peut régler une durée prédéterminée d'émission des rayons X par la source 16.

Le générateur 10 comporte également des moyens, par exemple un microprocesseur 12 en combinaison avec la minuterie 18, pour arrêter automatiquement l'émission des rayons X à la fin de la durée prédéterminée.

Le système d'acquisition 1 comporte également un capteur 30 intra buccal sensible aux rayons X, par exemple de type CMOS ou CCD.

Ce capteur intra buccal 30 est commandé par un module de commande 20 conforme à l'invention auquel il est relié par un fil 31.

Les images acquises par le capteur 30 sont également véhiculées dans ce fil 31 pour un premier traitement au niveau du module 20.

Dans l'exemple de réalisation décrit ici, le module 20 est contrôlé par un microprocesseur 21 adapté notamment à contrôler le capteur intra buccal 30, et à recevoir des données d'images numériques en provenance de ce capteur intra buccal par un port d'entrée/sortie 23.

Conformément à l'invention, le générateur 10 et le module de commande 20 comportent respectivement des moyens de communication 18, 22 par lesquels, notamment, le module 20 peut envoyer une commande de contrôle au générateur 10.

Dans une première variante de réalisation, ces moyens 18, 22 de communication sont des moyens de communication filaires.

En variante, ces moyens de communication peuvent être constitués par des moyens de communication sans fil, par exemple conformes à la norme BLUETOOTH.

Sur réception de cette commande de contrôle, le processeur 12 du générateur 10 interrompt l'émission, ou modifie la quantité d'émission, des rayons X par la source 16.

Si cette commande est reçue alors que la source 16 est inactive, le processeur 12 interdit toute émission ultérieure de rayons X par cette source 16, à moins d'une intervention volontaire manuelle de l'opérateur.

Dans le mode préféré de réalisation décrit ici, le module 20 selon l'invention comporte des moyens 28 de communication avec un système externe 40 constitué par exemple par un ordinateur personnel de type PC.

Cet ordinateur personnel 40 peut notamment être utilisé pour visualiser les images numériques acquises par le capteur intra buccal 30.

Dans le mode préféré de réalisation décrit ici, l'opérateur peut également utiliser le système distant 40 pour choisir un niveau de résolution (basse résolution BR, haute résolution HR) des images radiologiques acquises par le capteur 30, ce niveau de résolution BR, HR étant reçu par le module de commande 20 au moyen de son interface de communication 28.

Dans une variante non représentée ici, cette résolution BR, HR peut être programmée directement au niveau du module 20.

Dans le mode préféré de réalisation décrit ici, le processeur 21 du module de commande 20 est adapté à détecter un dysfonctionnement du capteur intra buccal 30, par exemple en lisant un registre du port d'entrée/sortie 23 prévu à cet effet.

Sur détection de ce dysfonctionnement, le processeur 21 envoie, au générateur 10 via ses moyens de communication 22, la commande de contrôle pour interrompre la génération de rayons X.

Dans le mode préféré de réalisation décrit ici, le processeur 21 est également adapté à déterminer le type T1 ou T2 du capteur intra buccal 30, par exemple en lisant un registre prévu à cet effet du port d'entrée/sortie 23.

Dans le mode préféré de réalisation décrit ici, le module de commande 30 comporte une mémoire non volatile 26 dans laquelle est mémorisée une table T comportant autant de colonnes que de types T1, T2 connus de capteurs 30 pouvant être commandés par le module 20, et autant de lignes que de résolutions BR, HR possibles pour les images de radiologie acquises par ce capteur.

En l'espèce, la table T comporte deux lignes et deux colonnes.

Cette table T mémorise quatre valeurs pour chaque couple (type T1, T2 de capteur/résolution HR, BR d'image) :
- une durée Di d'émission des rayons X par le générateur 10 pour le couple type/résolution correspondant ;
- un gain moyen GMi mesuré pour une séquence d'image précédemment acquise correspondant à ce couple type/résolution ;
- un gain Gi souhaité pour les images acquises pour ce couple type/résolution ; et
- un pas Pi d'ajustement de la durée Di lorsque le gain moyen GMi diffère du gain Gi souhaité.

Dans le mode préféré de réalisation décrit ici, pour les premières images acquises par un capteur intra buccal d'un type Ti, avec une résolution BR, HR, le processeur 21 du module de commande 20 lit dans la table T la durée Di souhaitée de diffusion des rayons X.

Le module de commande 20 comporte des moyens pour détecter le début de l'émission des rayons X par le générateur 10.

Ces moyens ne faisant pas partie de l'invention, ils ne seront pas détaillés ici. Ils peuvent notamment être constitués par tous les moyens de l'art antérieur cités dans le préambule de ce document, à savoir la réception d'une commande explicite en provenance du générateur 10, par exemple via le canal de communication 31, ou par des moyens de détection de type pastille sensible aux rayons X placée dans le capteur intra buccal 30.

Quoi qu'il en soit, le processeur 21 du module de commande 20 est adapté à déclencher une minuterie 24 pour mesurer la durée d'émission des rayons X à partir de la détection du début de cette émission.

Lorsque cette durée mesurée est égale à la durée Di mémorisée dans la table T, le processeur 21 envoie la commande de contrôle au générateur de rayons X 10, pour commander l'arrêt de l'émission des rayons X par le générateur 10.

Comme décrit précédemment, pour chaque image acquise, le processeur 21 met à jour le gain moyen GMi d'une séquence d'image acquise pour un couple type de capteur Ti résolution BR ou HR.

Quand un nombre suffisant d'images ont été acquises, lorsque le gain moyen GMi diffère du gain Gi souhaité pour ce type d'image, le processeur 21 ajuste la durée Ti avec un pas Pi. Par exemple, si le gain d'équilibre Gi souhaité est un gain de 6 dB, et si le gain moyen mesuré GMi pour les dernières images est un gain de 10 dB, alors le processeur 21 augmente la durée d'émission Di d'un pas Pi, par exemple 5 millisecondes.

En variante, le module 20 envoie une commande de contrôle au générateur 20, pour que celui-ci modifie, pour la prochaine acquisition, la quantité de rayons X émise conformément à cette quantité ajustée. L'homme du métier comprendra, en effet, que modifier le pas Pi revient à ajuster, à durée d'émission constante, la puissance d'émission des rayons X par le générateur.

## Revendications

1. Système d'acquisition d'image radiologique dentaire comportant :
- un générateur (10) de rayons X comportant des moyens (14) de déclenchement de l'émission des rayons X, une minuterie (18) permettant à un opérateur de régler une durée prédéterminée d'émission desdits rayons X, et des moyens (12) pour arrêter automatiquement ladite émission à l'issue de ladite durée prédéterminée ; et
- un capteur intra buccal (30) sensible aux rayons X, associé à un module (20) de commande dudit capteur (30), et adapté à acquérir l'image pendant ladite émission ;
- ledit module (20) de commande comportant des moyens pour démarrer ladite acquisition sur détection dudit déclenchement ; ledit système étant **caractérisé en ce que** :
- ledit module comporte des moyens (22) pour envoyer, selon au moins un critère prédéterminé, une commande de contrôle au générateur (10) pour interdire, arrêter, ou modifier la puissance de l'émission desdits rayons X avant la fin de ladite durée prédéterminée, de manière à contrôler la quantité de rayons X émise par le générateur ; **en ce que** :
- ledit générateur (10) comporte des moyens (18) de réception de ladite commande de contrôle, les moyens (12) d'arrêt du générateur (10) étant adaptés à interdire, arrêter ou modifier la puissance de ladite émission sur réception de ladite commande.

2. Système d'acquisition selon la revendication 1, **caractérisé en ce que** ledit module comporte des moyens (21, 23) pour détecter un dysfonctionnement dudit capteur (30) intra buccal, et (22) pour envoyer ladite commande de contrôle sur détection dudit dysfonctionnement.

3. Système d'acquisition selon la revendication 1 ou 2, **caractérisé en ce que** ledit module comporte des moyens (21, 23) pour déterminer le type (T1, T2) du capteur (30) intra buccal, des moyens pour déterminer une durée d'émission de rayons X en fonction de ce type, des moyens pour mesurer la durée d'émission de rayons X, et pour envoyer (22) ladite commande de contrôle à l'issue de ladite durée pour arrêter l'émission des rayons X par le générateur (10).

4. Système d'acquisition selon l'une quelconque des revendications 1 à 3, **caractérisé en ce que** ledit module (20) comporte des moyens (28) d'obtention d'un niveau de résolution (HR, BR) souhaité pour ladite image, des moyens (21) pour déterminer une durée d'émission de rayons X en fonction dudit niveau, des moyens (24) pour mesurer la durée d'émission de rayons X, et (22) pour envoyer ladite commande de contrôle à l'issue de ladite durée pour arrêter l'émission des rayons X par le générateur (10).

5. Système d'acquisition selon la revendication 4, **caractérisé en ce que** lesdits moyens d'obtention du niveau de résolution (HR, BR) sont des moyens de programmation dudit module (20) ou des moyens (28) de réception d'une information représentative dudit niveau (HR, BR) en provenance d'un dispositif distant (40).

6. Système d'acquisition selon l'une quelconque des revendications 1 à 5, **caractérisé en ce que** ledit module comporte des moyens pour mesurer le gain moyen (GMi) d'une séquence d'au moins une image précédemment acquise, des moyens pour déterminer une quantité (Qi) d'émission de rayons X ajustée en fonction dudit gain moyen (GMi) et d'un gain souhaité (Gi) prédéterminé, et des moyens (22) pour envoyer ladite commande de contrôle de manière à modifier la quantité de rayons X émise par ledit générateur conformément à ladite quantité ajustée.

7. Générateur de rayons X comportant des moyens (14) de déclenchement de l'émission des rayons X, une minuterie (18) permettant à un opérateur de régler une durée prédéterminée d'émission desdits rayons X, et des moyens (12) pour arrêter ladite émission à l'issue de ladite durée prédéterminée, ledit générateur (10) étant **caractérisé en ce qu'**il comporte des moyens (18) de réception d'une commande de contrôle en provenance d'un module (20) de commande d'un capteur (30) intra buccal sensible auxdits rayons, lesdits moyens d'arrêt (12) étant adaptés à interdire, arrêter ou modifier ladite quantité d'émission sur réception de ladite commande.

8. Module de commande d'un capteur (30) intra buccal sensible aux rayons X, **caractérisé en ce qu'**il comporte des moyens (22) pour envoyer, à un générateur (10) desdits rayons X, selon au moins un critère prédéterminé, une commande de contrôle pour interdire, arrêter ou modifier l'émission desdits rayons X par le générateur (10).

9. Module selon la revendication 8, **caractérisé en ce qu'**il comporte des moyens (21, 23) pour détecter un dysfonctionnement dudit capteur (30) intra buccal, et pour envoyer (22) ladite commande de contrôle sur détection dudit dysfonctionnement.

10. Module selon la revendication 8 ou 9, **caractérisé en ce qu'**il comporte des moyens (21, 23) pour détecter le type (T1, T2) du capteur (30) intra buccal, des moyens pour déterminer une durée d'émission de rayons X en fonction de ce type (T1, T2), des moyens pour mesurer la durée (Di) d'émission de rayons X, et (22) pour envoyer ladite commande de contrôle à l'issue de ladite durée pour arrêter l'émission des rayons X par le générateur (10).

11. Module selon l'une quelconque des revendications 8 à 10, **caractérisé en ce qu'**il comporte des moyens (28) d'obtention d'un niveau de résolution (HR, BR) souhaité pour ladite image, des moyens (21, T) pour déterminer une durée (Di) d'émission de rayons X en fonction dudit niveau (HR, BR), des moyens (24) pour mesurer la durée d'émission de rayons X, et pour envoyer ladite commande de contrôle à l'issue de ladite durée pour arrêter l'émission des rayons X par le générateur (10).

12. Module selon la revendication 11, **caractérisé en ce que** lesdits moyens d'obtention du niveau de résolution sont des moyens de programmation dudit module ou des moyens (28) de réception d'une information représentative dudit niveau (HR, BR) en provenance d'un dispositif distant (40).

13. Module selon l'une quelconque des revendications 8 à 12, **caractérisé en ce qu'**il comporte des moyens (21) pour mesurer le gain moyen (GMi) d'une séquence d'au moins une image précédemment acquise, des moyens pour déterminer une quantité (Qi) d'émission de rayons X ajustée en fonction dudit gain moyen (GMi) et d'un gain souhaité (Gi) prédéterminé, et des moyens (22) pour envoyer ladite commande de contrôle de manière à modifier la quantité de rayons X émise par ledit générateur conformément à ladite quantité ajustée.

## Claims

1. A dental X-ray image acquisition system comprising:
an X-ray generator (10) comprising means (14) for triggering the emission of X-rays, a timer (18) enabling an operator to set a predetermined emission duration for said X-rays, and means (12) for automatically stopping said emission at the end of said predetermined duration; and
• an intra-oral sensor (30) sensitive to X-rays, associated with a command module (20) for controlling said sensor (30), and adapted to acquire the image during said emission;
• said command module (20) including means for starting said acquisition on detecting said triggering; said system being **characterized in that**:
• said module includes means (22) for sending a command in application of at least one predetermined criterion to the generator (10) to prevent, stop, or modify the emission power of said X-rays before the end of said predetermined duration, so as to control the quantity of X-rays emitted by the generator;
• said generator (10) includes means (18) for receiving said command, the means (12) for stopping the generator (10) being adapted to prevent, stop, or modify the power of said emission on receiving said command.

2. An acquisition system according to claim 1, **characterized in that** said module includes means (21, 23) for detecting a malfunction of said intra-oral sensor (30), and means (22) for sending said command on detecting said malfunction.

3. An acquisition system according to claim 1 or claim 2, **characterized in that** said module includes means (21, 23) for determining the type (T1, T2) of the intra-oral sensor (30), means for determining an X-ray emission duration as a function of said type, means for measuring the duration of X-ray emission, and means (22) for sending said command at the end of said duration in order to stop the emission of X-rays by the generator (10).

4. An acquisition system according to any one of claims 1 to 3, **characterized in that** said module (20) includes means (28) for obtaining a level of resolution (HR, LR) desired for said image, means (21) for determining a duration for X-ray emission as a function of said level, means (24) for measuring the duration of X-ray emission, and means (22) for sending said command at the end of said duration in order to stop the emission of X-rays by the generator (10).

5. An acquisition system according to claim 4, **characterized in that** said means for obtaining the resolution level (HR, LR), are programming means of said module (20) or means (28) for receiving information representative of said level (HR, LR) coming from a remote device (40).

6. An acquisition system according to any one of claims 1 to 5, **characterized in that** said module includes means for measuring the mean gain (MGi) of a sequence of at least one previously acquired image, means for determining a quantity (Qi) of X-ray emission adjusted as a function of said mean gain (MGi) and as a function of a predetermined desired gain (Gi), and means (22) for sending said command in order to modify the quantity of X-rays emitted by said generator in compliance with said adjusted quantity.

7. An X-ray generator comprising means (14) for triggering the emission of X-rays, a timer (18) enabling an operator to adjust a predetermined duration for emission of said X-rays, and means (12) for stopping said emission at the end of said predetermined duration, said generator (10) being **characterized in that** it includes means (18) for receiving a command coming from a command module (20) of an intra-oral sensor (30) sensitive to said rays, said stop means (12) being adapted to prevent, stop, or modify said emission quantity on receiving said command.

8. A module for commanding an X-ray sensitive intra-oral sensor (30), the module being **characterized in that** it includes means (22) for sending a command to a generator (10) of said X-rays as a function of at least one predetermined criterion, the command serving to prevent, stop, or modify the emission of said X-rays by the generator (10).

9. A module according to claim 8, **characterized in that** it includes means (21, 23) for detecting a malfunction of said intra-oral sensor (30), and means (22) for sending said command on detection of said malfunction.

10. A module according to claim 8 or claim 9, **characterized in that** it includes means (21, 23) for detecting the type (T1, T2) of the intra-oral sensor (30), means for determining a duration for X-ray emission as a function of said type (T1, T2), means for measuring the duration (Di) of X-ray emission, and means (22) for sending said command at the end of said duration in order to stop the emission of X-rays by the generator (10).

11. A module according to any one of claims 8 to 10, **characterized in that** it includes means (28) for obtaining a level of resolution (HR, LR) desired for said image, means (21, 23) for determining a duration (Di) of X-ray emission as a function of said level (HR, LR), means (24) for measuring the duration of X-ray emission, and for sending said command at the end of said duration to stop the emission of X-rays by the generator (10).

12. A module according to claim 11, **characterized in that** said means for obtaining the level of resolution are means for programming said module or means (28) for receiving information representative of said level (HR, LR) coming from a remote device (40).

13. A module according to any one of claims 8 to 12, **characterized in that** it includes means (21) for measuring the mean gain (MGi) of a sequence of at least one previously acquired image, means for determining a quantity (Qi) of X-ray emission adjusted as a function of said mean gain (MGi) and of a predetermined desired gain (Gi), and means (22) for sending said command so as to modify the quantity of X-rays emitted by said generator in compliance with said adjusted quantity.

## Patentansprüche

1. System zur Aufnahme eines dentalen Röntgenbildes, umfassend:
- einen Röntgengenerator (10), der Mittel (14) zum Auslösen des Aussendens der Röntgenstrahlen, eine Zeitschaltuhr (18), die einer Bedienungsperson ermöglicht, eine vorbestimmte Röntgenstrahlenemissionsdauer einzustellen, sowie Mittel (12) zum automatischen Anhalten des Aussendens am Ende der vorbestimmten Dauer umfaßt, und
- einen röntgenstrahlenempfindlichen intraoralen Sensor (30), der mit einem Modul (20) zur Steuerung des Sensors (30) verbunden und dazu ausgelegt ist, das Bild während des Aussendens aufzunehmen,
- wobei das Steuerungsmodul (20) Mittel umfaßt, um die Aufnahme bei Erfassen der Auslösung zu starten, wobei das System **dadurch gekennzeichnet ist, daß**:
- das Modul Mittel (22) umfaßt, um nach wenigstens einem vorbestimmten Kriterium dem Generator (10) einen Steuerbefehl zu senden, um vor dem Ende der vorbestimmten Dauer das Aussenden der Röntgenstrahlen zu unterbinden, zu stoppen oder die Leistung des Aussendens der Röntgenstrahlen zu ändern, um die durch den Generator ausgesandte Röntgenstrahlenmenge zu steuern,
- der Generator (10) Mittel (18) zum Empfangen des Steuerbefehls umfaßt, wobei die Mittel (12) zum Abstellen des Generators (10) dazu ausgelegt sind, bei Empfang des Befehls das Aussenden zu unterbinden, zu stoppen oder die Leistung des Aussendens zu ändern.

2. Aufnahmesystem nach Anspruch 1, **dadurch gekennzeichnet, daß** das Modul Mittel (21, 23) zum Erfassen einer Fehlfunktion des intraoralen Sensors (30) sowie (22) zum Senden des Steuerbefehls bei Erfassen der Fehlfunktion umfaßt.

3. Aufnahmesystem nach Anspruch 1 oder 2, **dadurch gekennzeichnet, daß** das Modul Mittel (21, 23) zum Bestimmen des Typs (T1, T2) des intraoralen Sensors (30), Mittel zum Bestimmen einer Röntgenstrahlenemissionsdauer in Abhängigkeit dieses Typs, Mittel zum Messen der Röntgenstrahlenemissionsdauer und zum Senden (22) des Steuerbefehls am Ende der Dauer, um das Aussenden der Röntgenstrahlen durch den Generator (10) zu stoppen, umfaßt.

4. Aufnahmesystem nach einem der Ansprüche 1 bis 3, **dadurch gekennzeichnet, daß** das Modul (20) Mittel (28) zum Erhalten eines für das Bild gewünschten Auflösungsgrades (HR, BR), Mittel (21) zum Bestimmen einer Röntgenstrahlenemissionsdauer in Abhängigkeit des Grades, Mittel (24) zum Messen der Röntgenstrahlenemissionsdauer sowie (22) zum Senden des Steuerbefehls am Ende der Dauer, um das Aussenden der Röntgenstrahlen durch den Generator (10) zu stoppen, umfaßt.

5. Aufnahmesystem nach Anspruch 4, **dadurch gekennzeichnet, daß** die Mittel zum Erhalten des Auflösungsgrades (HR, BR) Programmierungsmittel des Moduls (20) oder Mittel (28) zum Empfangen einer für den Grad (HR, BR) repräsentativen Information von einer entfernten Vorrichtung (40) sind.

6. Aufnahmesystem nach einem der Ansprüche 1 bis 5, **dadurch gekennzeichnet, daß** das Modul Mittel zum Messen der durchschnittlichen Verstärkung (GMi) einer Sequenz von wenigstens einem zuvor aufgenommenen Bild, Mittel zum Bestimmen einer Röntgenstrahlenemissionsmenge (Qi), die in Abhängigkeit der durchschnittlichen Verstärkung (GMi) und einer vorbestimmten gewünschten Verstärkung (Gi) eingestellt wird, sowie Mittel (22) zum Senden des Steuerbefehls umfaßt, um die durch den Generator ausgesandte Röntgenstrahlenmenge entsprechend der eingestellten Menge zu ändern.

7. Röntgengenerator, umfassend Mittel (14) zum Auslösen des Aussendens der Röntgenstrahlen, eine Zeitschaltuhr (18), die einer Bedienungsperson ermöglicht, eine vorbestimmte Röntgenstrahlenemissionsdauer einzustellen, sowie Mittel (12) zum Anhalten des Aussendens am Ende der vorbestimmten Dauer, wobei der Generator (10) **dadurch gekennzeichnet ist, daß** er Mittel (18) zum Empfangen eines Steuerbefehls von einem Modul (20) zum Steuern eines gegenüber den Strahlen empfindlichen intraoralen Sensors (30) umfaßt, wobei die Abstellmittel (12) dazu ausgelegt sind, bei Empfang des Befehls das Aussenden zu unterbinden, zu stoppen oder die Emissionsmenge zu ändern.

8. Modul zum Steuern eines röntgenstrahlenempfindlichen intraoralen Sensors (30), **dadurch gekennzeichnet, daß** es Mittel (22) umfaßt, um nach wenigstens einem vorbestimmten Kriterium einem Röntgengenerator (10) einen Steuerbefehl zu senden, um das Aussenden der Röntgenstrahlen durch den Generator (10) zu unterbinden, zu stoppen oder zu ändern.

9. Modul nach Anspruch 8, **dadurch gekennzeichnet, daß** es Mittel (21, 23) zum Erfassen einer Fehlfunktion des intraoralen Sensors (30) sowie zum Senden (22) des Steuerbefehls bei Erfassen der Fehlfunktion umfaßt.

10. Modul nach Anspruch 8 oder 9, **dadurch gekennzeichnet, daß** es Mittel (21, 23) zum Erfassen des Typs (T1, T2) des intraoralen Sensors (30), Mittel zum Bestimmen einer Röntgenstrahlenemissionsdauer in Abhängigkeit dieses Typs (T1, T2), Mittel zum Messen der Röntgenstrahlenemissionsdauer (Di) sowie (22) zum Senden des Steuerbefehls am Ende der Dauer, um das Aussenden der Röntgenstrahlen durch den Generator (10) zu stoppen, umfaßt.

11. Modul nach einem der Ansprüche 8 bis 10, **dadurch gekennzeichnet, daß** es Mittel (28) zum Erhalten eines für das Bild gewünschten Auflösungsgrades (HR, BR), Mittel (21, T) zum Bestimmen einer Röntgenstrahlenemissionsdauer (Di) in Abhängigkeit des Grades (HR, BR), Mittel (24) zum Messen der Röntgenstrahlenemissionsdauer und zum Senden des Steuerbefehls am Ende der Dauer, um das Aussenden der Röntgenstrahlen durch den Generator (10) zu stoppen, umfaßt.

12. Modul nach Anspruch 11, **dadurch gekennzeichnet, daß** die Mittel zum Erhalten des Auflösungsgrades Programmierungsmittel des Moduls oder Mittel (28) zum Empfangen einer für den Grad (HR, BR) repräsentativen Information von einer entfernten Vorrichtung (40) sind.

13. Modul nach einem der Ansprüche 8 bis 12, **dadurch gekennzeichnet, daß** es Mittel (21) zum Messen der durchschnittlichen Verstärkung (GMi) einer Sequenz von wenigstens einem zuvor aufgenommenen Bild, Mittel zum Bestimmen einer Röntgenstrahlenemissionsmenge (Qi), die in Abhängigkeit der durchschnittlichen Verstärkung (GMi) und einer vorbestimmten gewünschten Verstärkung (Gi) eingestellt wird, sowie Mittel (22) zum Senden des Steuerbefehls umfaßt, um die durch den Generator ausgesandte Röntgenstrahlenmenge entsprechend der eingestellten Menge zu ändern.
